# EUROPÄISCHE PATENTSCHRIFT

(11) **EP 3 090 626 B1**
(45) Veröffentlichungstag und Bekanntmachung des Hinweises auf die Patenterteilung: **13.03.2019**
(21) Anmeldenummer: 15001387.8
(22) Anmeldetag: 08.05.2015
(51) Int. Cl.: A01G 33/00, C12N 1/12

(54) **VERFAHREN ZUR HERSTELLUNG EINER ALGENFRISCHMASSE**
METHOD FOR PRODUCING A FRESH ALGAE MASS
PROCÉDÉ DE PRÉPARATION À PARTIR D'ALGUES FRAÎCHES

(43) Veröffentlichungstag der Anmeldung: 09.11.2016
(73) Patentinhaber: Ökotec-Anlagenbau GmbH, 04808 Thallwitz (DE)
(72) Erfinder: WILHELM, Gerhard, D-04808 Thallwitz (DE)
(74) Vertreter: Müller, Volkmar

(56) Entgegenhaltungen:
- WO-A1-2011/026482
- DE-U1-202014 006 166
- DE-U1-202014 006 168

## Beschreibung

Die Erfindung betrifft ein Verfahren zur Herstellung einer Algenfrischmasse, wobei nach der Ernte unter Nutzung eines Filtrationsverfahrens frisch geerntetes flüssiges Algensubstrat mit einem Trockensubstanzgehalt von 5 bis 7 % und einem pH-Wert zwischen 9,2 bis 9,6 vorliegt.
Aus der WO 2011/026482 A1 ist ein Verfahren zum Ernten von Algen aus einer Algensuspension bekannt. Bei diesem Verfahren erfolgt eine Aufkonzentration der Algensuspension mittels eines Membranfilters, wobei dabei zumindest eine massive Schädigung der Algen erfolgt. Hinweise zu einer nachfolgenden Lagerung der Algensuspension werden nicht gegeben.

In der DE 20 2014 006 168 U1 wird vom Anmelder, der Firma Öcotec-Anlagenbau GmbH, ein Wickel mit lebenden Spirulina-Algen beschrieben.

In der DE 20 2014 006 168 U1 wird vom Anmelder, der Firma Öcotec-Anlagenbau GmbH, eine Anlage zur Aufzucht und Reproduktion von Spirulina-Algen beschrieben.
Die Ernte, beispielsweise einer Schwimmdecke von flüssigem Algensubstrat, kann durch ein mehrstufiges hydrostatisches Filtrationsverfahren erfolgen. Dabei kann ein dünnflüssiges Algensubstrat im Sieb zurückbleiben. Dieses wird nicht weiter aufkonzentriert; wobei der Trockensubstanzgehalt (TS-Gehalt) bei ca. 6 % liegt. Die Bestimmung des Trockensubstanzgehalts kann in Anlehnung an die DIN 12880 bzw. DIN 12879 erfolgen.
Ein solches Verfahren ist beispielsweise in der DE 10 2014 011 317.5 grundsätzlich beschrieben. Dieses Schwimmschicht-Ernteverfahren ist nur bei Beckenreaktoren möglich, wobei die konzentrierte Algenbiomasse auf dem Medium schwimmend, über ein mobiles Wehr in einen Sammelbehälter abfließt.
Diese schonende Ernte und das hydrostatische Filtrationsverfahren schädigen die einzelne Algenzelle wenig oder gar nicht, d.h. die Trichom-Struktur bleibt weitgehend intakt und damit teilungsfähig.
Der Vorteil in diesem Ernteregime besteht darin, dass sich durch die oberflächige Ernte des flüssigen Algensubstrats das Biosystem der Algenkultur schnell wieder stabilisiert und weitere diesbezügliche Ernten, die regelmäßig in kontinuierlichen zeitlichen Abständen erfolgen, gewährleistet sind.
Dieses Verfahren kann beispielsweise unter Verwendung einer Anlage zur Aufzucht und Reproduktion von Spirulina-Algen, beschrieben in der DE 20 2014 006 168, realisierbar werden.

Nach der Ernte setzen in der frischen Biomasse, d. h. dem frisch geernteten flüssigen Algensubstrat, sehr rasch verschiedene chemische und mikrobielle Abbauprozesse ein, die diese verunreinigen und qualitativ schädigen können. Das flüssige Algensubstrat enthält wunschgemäß, zumindest teilweise, lebende Algen, welche in diesem Zustand möglichst belassen werden sollen.

Eine Verarbeitung des flüssigen Algensubstrates kann oft nicht zeitnah erfolgen oder soll gezielt erst später erfolgen, beispielsweise in dem Fall, wobei ein Chargenbetrieb vorliegt.

Um diese unerwünschten Prozesse weitgehend zu reduzieren oder zu unterbinden, sind geeignete Verfahren zur Konservierung und Lagerung des flüssigen Algensubstrates erforderlich, welche aus dem Stand der Technik bisher nicht bekannt sind, obwohl ein diesbezüglicher Bedarf besteht.

Aufgabe der Erfindung ist es, ein solches Verfahren zur Konservierung und Lagerung des frisch geernteten flüssigen Algensubstrates bereit zu stellen, welches möglichst schonend erfolgt, so dass der Anteil lebender Algen möglichst hoch bleibt. Dieses Verfahren soll insbesondere in technologischer Hinsicht auf die nachfolgende Verarbeitung des Algensubstrates zu einem Algenprodukt abgestimmt sein.
Die Aufgabe der Erfindung wird durch ein Verfahren mit den Merkmalen gemäß Anspruch 1 gelöst.
Erfindungswesentlich ist, dass das frisch geerntete flüssige Algensubstrat und/oder das bis zu 48 Stunden gelagerte flüssige Algensubstrat zu einer Algenfrischmasse verarbeitet werden, wobei das flüssige Algensubstrat auf einen Trockensubstanzgehalt von 10 bis 15% entwässert und nachfolgend der pH-Wert zwischen 8,5 bis 8,8 eingestellt wird.

Dieses Verfahren ist u. a. der Art ausgelegt, das in möglichst technisch und technologisch einfachen Art und Weise eine Lagerung und Vorbereitung des frisch geernteten flüssigen Algensubstrat für die nachfolgende Verarbeitung, beispielsweise zu Algenprodukten, im Rahmen einer industriellen Herstellung ermöglich ist. Dies erfordert regelmäßig eine reproduzierbare und technologisch optimierte Prozessführung bei dieser industriellen Herstellung.

Die erfindungsgemäße Entwässerung des frisch geernteten flüssigen Algensubstrats ist dabei wesentlich.
Die Entwässerung des frisch geernteten flüssigen Algensubstrats auf einen definierten Trockenmasse-Bereich von 11 bis 13 % (TM) ist insbesondere geeignet, um den Produktionsprozess zu vereinfachen. So ist beispielsweise der konstante Einsatz von Stoffzusätzen, wie Lößerde und Cellulose, ermöglicht.
In diesem ausgewählten Trockenmasse-Bereich besitzt das Algensubstrat bereits eine pastöse Gelstruktur (= Gel zwischen äußeren Hüllschichten der Trichome), welche eine solche Konsistenz besitzt, welche man als fast stichfest bezeichnen kann. Außerdem weist diese solche Eigenschaften auf, so dass diese gute Mischungseigenschaften besitzt; welche bei der Herstellung von Algenprodukten erforderlich ist. Dies ist ein entscheidender Vorteil für die weitere Verarbeitung der Frischmasse (FM).

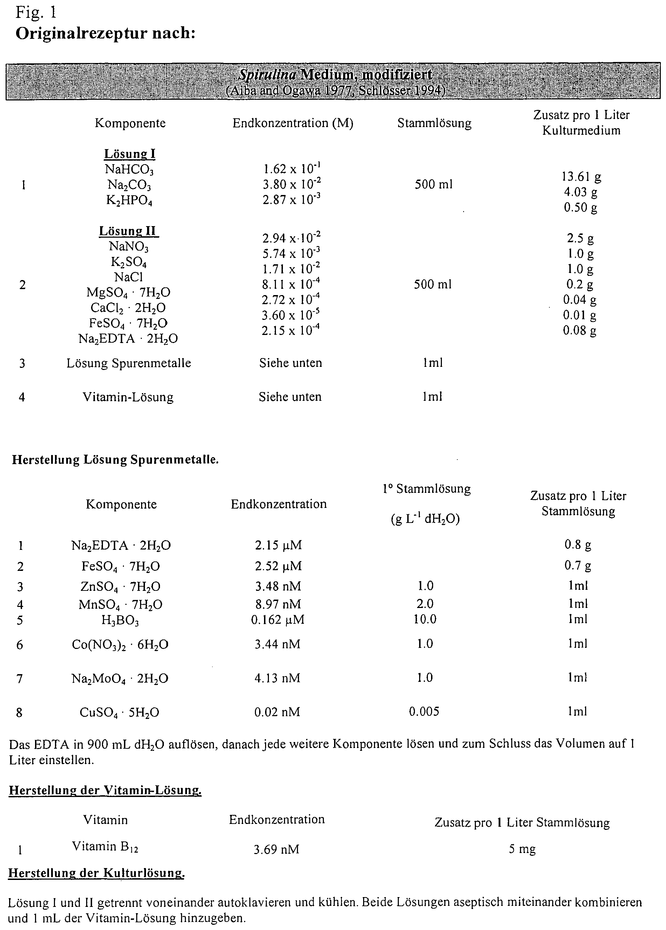

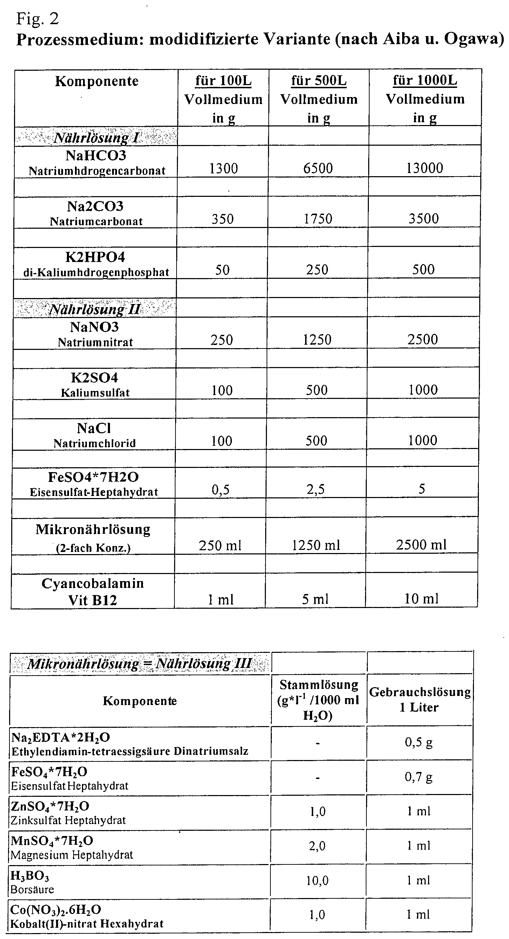

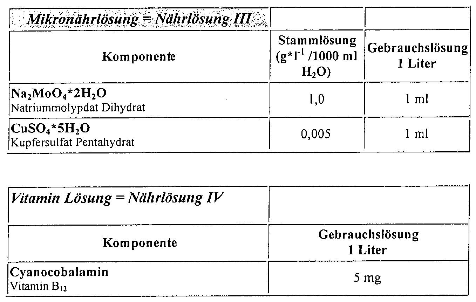

Die erfindungsgemäße Einstellung eines vorbestimmten pH-Wert-Bereiches der Frischalgenmasse, nachfolgend zum Verfahrensschritt der Entwässerung, ist erfindungswesentlich.
Dabei sind solche chemischen Stoffe grundsätzlich einsetzbar, welche insbesondere den pH-Wert senken, zumindest eine Konservierung unterstützen, im Rahmen der nachfolgenden Verarbeitung nicht unzweckmäßig sind und/oder keine Schädigung der lebenden Algen bewirken.
Die abhängigen Ansprüche 2 bis 5 enthalten vorteilhafte Ausgestaltungen der Erfindung ohne diese damit zu begrenzen.
Bevorzugt ist, dass nach der Ernte eine Lagerung des flüssigen Algensubstrates bis zu 48 Stunden im Prozessmedium unter Prozessbedingungen erfolgt.
Ein Prozessmedium im Sinne der Erfindung ist insbesondere charakterisiert durch eine übliche Zusammensetzung aus verschiedenen Nährsalzen, von Makro-bzw. Mikronährstoffen und Spurenelementen.
Dieses Prozessmedium kann das bisher im Verfahren eingesetzte sein; ein frisches, welches dann in üblicher Weise das bisher im Verfahren eingesetzte ersetzt oder ein Gemisch aus bisher im Verfahren eingesetzten und frischen Prozessmedium.
Ein solches Prozessmedium (Fig. 2) kann beispielsweise auf einem Medium von Aiba und Ogawa (J. Gen. Microbiol. (1977), 102, 179-182) (Fig.1), basieren.

Prozessbedingungen im Sinne der Erfindung sind übliche Parameter bei der Aufzucht von Algenkulturen, insbesondere von Spirulina. Algenkulturen, insbesondere hinsichtlich Temperatur (25°C), Rührzeiten, Beleuchtungsstärke und Lichtspektrum. Diese Parameter sind auf die jeweiligen Prozessschritte abgestimmt und justiert.
Dazu zählen beispielsweise:
Beheizbare Beckenreaktoren (Heizung mit Wärmepumpe Luft-Luft) sowie automatische Be - und Entlüftung des Gewächshauses, automatisch gesteuerte bzw. dem Prozess angepasste Rührzeiten und manuelle Steuerung der Beleuchtung (Verschattung) sorgen für eine relativ konstante Prozesstemperatur.
Natrium-Hochdruckdampflampen (450 Watt) liefern in den lichtarmen Monaten fehlende Lichtspektren. Nicht gesamte die Globalstrahlung, sondern nur der sichtbare Bereich von 400 bis 780nm ist für die photosynthetisch aktiven Organismen verwertbar, auch PAR (engl.: Photosynthetically Active Radiation, kurz PAR oder PhAR) genannt.
Es ist der Bereich im Spektrum der Sonnenstrahlung, von 400 bis 700 nm in W/m² angegeben. Dieser Bereich deckt sich weitgehend mit dem Bereich der für Menschen sichtbaren Strahlung (380-780nm), welcher etwa 50 % der Globalstrahlung ausmacht.
Die photosynthetisch aktive Strahlung setzt sich aus Photonen mit sehr unterschiedlicher Energie zusammen (blau: energiereich, rot: energiearm). Wegen der direkten stöchiometrischen Beziehung zwischen absorbierten Photonen (im Bereich von 400 bis 700nm) und der photosynthetischen CO₂-Bindung wurde die Photonenstromdichte (engl. Photosynthetically Active Photon Flux Density, PPFD oder kurz PFD) in der Biologie zum Standard. Sie wird, im Gegensatz zum PAR, in µmol/(m²s) gemessen.

Die qualitativ besten Ernteergebnisse liefert die Einstrahlung mit hohem Anteil gelbroter Wellenlänge. Überwiegt die energiereiche blau-grüne Strahlung, so muss die daraus resultierende hohe Photonenstromdichte, die auf die Oberfläche des Beckenreaktors trifft, reduziert werden. Mit manuell gesteuerter Verschattung kann zumindest größtenteils schädigende Einstrahlung von der Kultur abgehalten werden. Auch eine erhöhte Rührfrequenz reduziert die Belichtungszeit der einzelnen Alge und verhindert eine stärkere Schädigung des Algentrichoms.

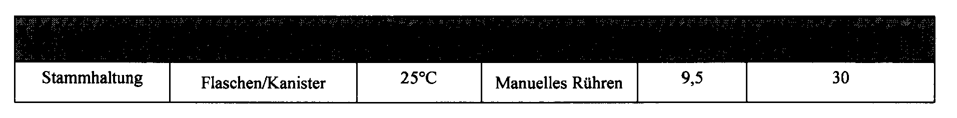

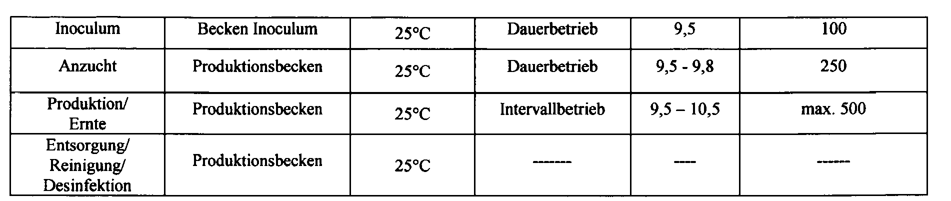

Eine solche Lagerung im "filtrierten" Prozessmedium bei solchen normalen Prozessbedingungen ist nur möglich, weil die Algenzelle des flüssigen Algensubstrates auch nach der Ernte, zumindest teilweise, lebens- und damit teilungsfähig bleibt.
Vorteilhaft ist dabei, dass das geerntete flüssige Algensubstrat unter den gewohnten Prozessbedingungen im Medium "lagert", wenigen Veränderungen ausgesetzt ist und dadurch noch nach der Ernte weitere Biomasse produzieren kann.
Diese Verfahrensweise ist insbesondere in den Monaten mit gemäßigten im Gewächshaus-Temperaturen praktizierbar.

Eine kurzzeitige Lagerung des flüssigen Algensubstrates, d. h. bis zu 48 Stunden, kann ohne eine gesonderte chemische Konservierung erfolgen.
Dies ist überraschend deshalb möglich, weil der erfindungsgemäß ausgewählte Bereich des pH-Wertes, nämlich ein pH -Wert zwischen 9,5 - 10,5, ein stark basisches Medium (mit einem pH -Wert von 9,5 - 10,5), bewirkt, welches eine übliche konservierende Wirkung besitzt. Damit besitzt das Prozessmedium gleichzeitig die Funktionalität eines Lager- und Nährmediums.

Dieses sog. "Zwischenlagerungs-Verfahren" von max. 2 Tagen eignet sich beispielsweise hervorragend für die tägliche Ernte am Wochenende, wobei das durch die Ernte gewonnene frisch geerntete flüssige Algensubstrat schonend gelagert und somit erst am (an) folgenden Werktag(en) verarbeitet wird (werden) braucht.
Es wurde somit in technisch und technologisch einfachster Art und Weise ein überraschend einfaches, unkompliziertes und kostengünstiges Lagerungsverfahren, u. a. mit einem geringen Zeitaufwand und Gerätebedarf, bereitgestellt.

Erfindungswesentlich ist weiterhin, dass eine Lagerung des flüssigen Algensubstrates im Prozessmedium bei einer Temperatur von 2 bis 4°C und im Dunkeln erfolgt.

Untersuchungen haben ergeben, dass eine Lagerung des flüssigen Algensubstrates im Prozessmedium bei einer Temperatur von 2 bis 4°C und im Dunkeln zu einer signifikanten Veränderung der Struktur des flüssigen Algensubstrates führen kann.

Bevorzugt ist weiterhin, dass der pH-Wert des flüssigen Algensubstrates durch Zugabe einer Ascorbinsäure-Lösung, bevorzugt einer 20 % -igen Ascorbinsäure-Lösung, auf einen Wert zwischen 8,2 bis 8,8 eingestellt wird.
Ascorbinsäure besitzt im Sinne der Erfindung gewünschte Eigenschaften eines Konservierungsstoffs und einer Säure.
Ascorbinsäure (Vitamin C), eignet sich für die Konservierung von Algenbiomasse, da es ein natürliches Konservierungsmittel ist, welches die schnelle Vermehrung von Mikroorganismen hemmt, welche sofort nach der Ernte einsetzt und zu verhindern ist. Ascorbinsäure ist ein Zusatzstoff, der sowohl in der Lebensmittelindustrie (E 300) als auch für homöopathische bzw. kosmetische Anwendungen bereits eingesetzt wird. Beispielsweise ist die Verwendung von Ascorbinsäure aus der Homöopathie im Zusammenhang mit der Prophylaxe mit hochkonzentriertem Vitamin. C Infusionen bekannt.
Ascorbinsäure verursacht positive Wirkung auf die Haut: stimuliert den Bindegewebsstoffwechsel, wirkt regenerierend und heilend auf Bindegewebsschäden. Ascorbinsäure (Vitamin C) neutralisiert außerdem den spezifischen Eigengeruch der Alge im Endprodukt, welcher mit zunehmender Lagerungsdauer oft intensiver wird. Ascorbinsäure besitzt außerdem einen Säurecharakter und eignet sich zum Senken des pH-Wertes in der Algen-Frischmasse (von ca. 9,5) auf 8,8 bis 8,5.

Bevorzugt ist dabei die Anwendung einer 20 % - igen Ascorbinsäure-Lösung gelöst in Wasser (max. sind 333 g Ascorbinsäure/Liter löslich).

Bevorzugt ist, dass die Ausgangsmaterialien des Algenwickels zumindest eine Algenfrischmasse, Lösserde und Cellulose sind, welche zu einer Wickelmasse verarbeitet werden.

Die beiden Zusatzstoffe, nämlich Lösserde, auch Heilerde genannt, und Cellulose, bilden zusammen mit der äußeren Hülle der Algenzelle die eigentliche Gelstruktur des Algenwickels. Es ist ein disperses System, bestehend aus den festen Komponenten (Algen, Heilerde, Cellulose), die ein schwammartiges, dreidimensionales Netzwerk bilden, dessen Poren durch Wasser ausgefüllt sind und dadurch ist die flüssige Komponente in der festen immobilisiert.

Cellulose, beispielsweise als Lebensmittelzusatzstoff E 460, ist ein für den Lebensmittels-Bereich zugelassenes Bindemittel und eignet sich hervorragend für die Stabilisierung der Wickelmasse.
Cellulose wird bevorzugt in der kleinsten verfügbaren Körnung (Korngröße 30 µm) eingesetzt; soll zum einen möglichst viel Wasser binden, um die Wickelmasse bezüglich ihrer Konsistenz durch Erhöhung der Viskosität weiter zu stabilisieren, und zum anderen ein in sich homogenes bzw. stabiles Algenprodukt garantieren (geringer Wasseraustritt bei der Anwendung und verschiedenen Lagerungs-Verfahren).

Lösserde besteht aus mineralstoffreichen Löss-Partikeln. Diese Löss-Partikel haben generell eine große Teilchen-Oberfläche, an der Wasser haften kann, wobei die Speicherfähigkeit umso größer ist, je feiner die Löss-Partikel sind und je größer dementsprechend die Teilchenoberfläche ist.
Löss fühlt sich auch noch in dem Fall, wo er verhältnismäßig viel Wasser enthält, als trockenes Gut an und kann daher gut mit der sehr wasserhaltigen Algen-Biomasse gemischt werden, ohne dass diese ihre gewünschten Eigenschaften verliert.

Löss kann in mittelfeiner Körnung, beispielsweise bei einer äußeren Anwendungen, und in geprüfter/keimfreier Qualität entsprechend dem deutschen Lebensmittelgesetz eingesetzt werden.

Beide Zusatzstoffe wirken während Lagerphase auch prozessstabilisierend, weil sie freies Wasser in der Biomasse binden und so das Wachstumsmilieu für andere Bakterien reduzieren, d.h. für eine Lagerdauer bis zu maximal sieben Tagen, kann das Wachstum unerwünschter Mikroorganismen bei kühler Lagerung (< 5 °C) begrenzt werden.
Der kompakte Strukturverband des Algenwickels bleibt, wie funktionsbedingt erforderlich, sowohl während als auch nach der Anwendung bestehen, d.h. auch im Falle von Austrocknungen des Algenwickels wird die Gelstruktur nicht oder zumindest nicht signifikant zerstört.

Die Herstellung der Wickelmasse für einen Algenwickel kann zumindest auf der Basis der Ausgangsmaterialien: Algenfrischmasse, Lösserde und Cellulose erfolgen. Der Algenwickel besitzt einen pH-Wert zwischen 8,0 und 8,3.

Weitere Merkmale, Eigenschaften und Vorteile der vorliegenden Erfindung ergeben sich aus der nachfolgenden Beschreibung von Ausführungsbeispielen.

### Ausführungsbeispiel 1

Verfahrensablauf zur Herstellung einer Algenfrischmasse (ohne Lagerung).

### Schritt 1

Die Ernte einer auf der Nährlösung schwimmenden Algen-Schwimmdecke des Beckenreaktors, erfolgt durch ein mehrstufiges hydrostatisches Filtrationsverfahren. Nach dieser Filtration bleibt ein dünnflüssiges Algensubstrat im Sieb zurück, welches einen Trockensubstratgehalt von ca. 6 % besitzt und dessen pH-Wert ca. 9,2 beträgt (TS-Bestimmung erfolgt dabei in Anlehnung nach DIN 12880 bzw. 12879 in üblicher Art und Weise).

Das gewonnene dünnflüssige Algensubstrat enthält Spirulina-Algen unterschiedlicher Länge, welche insbesondere durch jeweils unterschiedliche Wachstumsphasen der Spirulina-Algen verursacht sind.
Außerdem können Fremdalgen, d. h. die keine Spirulina-Algen sind, im gewonnenen Algensubstrat enthalten sein, welche regelmäßig nicht weiter zu einem erfindungsgemäßen Algenprodukt behandelt werden sollen.
Während der Ernte des lebenden dünnflüssigen Algensubstrats kann eine Abtrennung von flüssigem Algensubstrat, welches insbesondere Spirulina-Algen enthält, mit einer Ausdehnung von zumindest 30 µm vom Gemisch aus anderen kleinen Blaualgen und Fremdalgen mit jeweils einer maximalen Ausdehnung von 10 µm erfolgen.

### Schritt 2

Nach dem Schritt 1 erfolgt innerhalb von ca. 3 Stunden eine schonende Entwässerung des gewonnenen Algensubstrats auf einen Trockensubstratgehalt von ca. 11 %.
Es hat sich herausgestellt, dass eine Algenfrischmasse mit konstantem Trockensubstratgehalt, hier ca. 11 % Trockensubstratgehalt, die weiteren Verarbeitungsprozesse erleichtert, indem manuelle Produktionsabschnitte teilweise durch gewisse Automatismen, ersetzbar sind. Daraus resultieren Arbeitseinsparungen und eine höhere Produktivität.
Im Anschluss an die erfolgte Entwässerung wird der pH-Wert der Algenfrischmasse auf einen Wert von ca. 8,5 eingestellt.
Der pH-Wert des flüssigen Algensubstrates (2) wird durch Zugabe einer 20 % -igen Ascorbinsäure-Lösung in üblicher Art und Weise auf einen Wert von ca. 8,5 eingestellt.

Nunmehr liegt eine Algenfrischmasse mit einem Trockensubstratgehalt von ca. 11 % und einem pH-Wert von ca. 8,5 vor.

### Ausführungsbeispiel 2

Verfahrensablauf zur Herstellung einer Algenfrischmasse (mit Lagerung)

Die Ernte einer Algen-Schwimmdecke aus dem Beckenreaktor erfolgt analog Schritt 1 durch ein mehrstufiges hydrostatisches Filtrationsverfahren.
Nach dieser Filtration bleibt ein dünnflüssiges Algensubstrat, beispielsweise im Sieb, zurück, welches einen Trockensubstratgehalt von ca. 6 % besitzt (TS-Bestimmung erfolgt dabei in Anlehnung nach DIN 12880 bzw. 12879) und dessen pH-Wert zwischen ca. 9,2 beträgt. Das gewonnene dünnflüssige Algensubstrat enthält Spirulina-Algen unterschiedlicher Ausdehnung, welche insbesondere durch jeweils unterschiedliche Wachstumsphasen der Spirulina-Algen verursacht sind.
Außerdem können Fremdalgen im gewonnenen Algensubstrat enthalten sein, welche regelmäßig nicht weiter zu einem erfindungsgemäßen Algenprodukt behandelt werden sollen.
Während der Ernte des lebenden dünnflüssigen Algensubstrats kann eine Abtrennung von flüssigem Algensubstrat 2, welches insbesondere Spirulina-Algen enthält, mit einer Ausdehnung von zumindest 30 µm von dem Gemisch aus anderen kleinen Blaualgen und Fremdalgen mit jeweils einer maximalen Ausdehnung von 10 µm erfolgen.

Nach dem Schritt 1 erfolgt innerhalb von bis zu 48 Stunden eine Lagerung des flüssigen Algensubstrates im Prozessmedium unter Prozessbedingungen.

Dieses Prozessmedium ist ein übliches Nährmedium, welches für lebende Spirulina-Algen geeignet ist. Dieses Nährmedium enthält eine übliche Nährlösung, welche insbesondere Nährsalze, in Wasser gelöst, enthält.

Die Prozessbedingungen werden insbesondere durch die Kultur-Temperatur von 25 °C sowie den Gewächshaus-Bedingungen mit wechselnder natürlicher Lichteinstrahlung hinsichtlich Strahlenspektrum und Pflanzen verwertbarer Lichtenergie.

Dabei wird Algensubstrat mit einem Trockensubstratgehalt von ca. 6 % gelagert, um Schädigungen der Trichome des dünnflüssigen Algensubstrats so gering wie möglich zu halten. Durch diese Lagerung, welche bezogen auf den Gesamtprozess eine Zwischenlagerung ist, des dünnflüssigen Algensubstrats im Nährmedium bleiben die Lebens- und Wachstumsbedingungen erhalten, welche für eine Weiterverarbeitung zu einem erfindungsgemäßen Algenprodukt notwendig sind.

Diese Lagerung ist durch die Anwendung der nachfolgenden alternativen Schritte grundsätzlich möglich:
Schritt 3₁
   Bei Prozessbedingungen: 25-30°C und natürlicher Beleuchtung (Biomassezuwachs) oder
Schritt 3₂
   Bei ca. 2- 4°C ohne Beleuchtung (ohne Biomassezuwachs).

Nach dem Schritt 3₁ oder 3₂ erfolgt innerhalb von ca. 3 Stunden eine schonende Entwässerung des gewonnenen Algensubstrats auf einen Trockensubstratgehalt von ca. 11 %.
Es hat sich herausgestellt, dass eine Algenfrischmasse mit konstantem Trockensubstratgehalt, hier ca. 11 % Trockensubstratgehalt, die weiteren Verarbeitungsprozesse erleichtert, indem manuelle Produktionsabschnitte teilweise durch gewisse Automatismen ersetzbar sind. Daraus resultieren Arbeitseinsparungen und eine höhere Produktivität.
Im Anschluss an die erfolgte Entwässerung wird der pH-Wert der Algenfrischmasse auf einen Wert von ca. 8,2 eingestellt.
Der pH-Wert des flüssigen Algensubstrates 2 wird durch Zugabe einer 20 % -igen Ascorbinsäure-Lösung in üblicher Art und Weise auf einen Wert von ca. 8,2 eingestellt. Nunmehr liegt eine Algenfrischmasse mit einem Trockensubstratgehalt von ca. 11 % und einem pH-Wert von ca. 8,2 in analoger Weise zum Schritt 2 vor.

### Ausführungsbeispiel 3

### Algenprodukt, hier Algenwickel

Eine Algenfrischmasse erzielbar gemäß den Schritten 1 und 2(Ausführungsbeispiel 1) oder des Schrittes 3(Ausführungsbeispiel 2), insbesondere mit einem Trockensubstratgehalt von ca. 11 % und einem pH-Wert von ca. 8,5, ist u. a. das Ausgangsmaterial für einen Algenwickel bzw. einer Wickelmasse.

Der pH-Wert des flüssigen Algensubstrates zu Beginn des Schrittes 1 liegt zwischen 9,2 - 9,5, wobei Schwankungen durch einen unterschiedlichen Entwässerungsgrad verursacht sein können.

Durch Zugabe, zur Algenfrischmasse mit einem pH-Wert von max. 8,5, von Löss-Erde Pulver und Cellulose-Pulver wird die Grundmasse der Wickelmasse erhalten, welche durch weitere Einsatzstoffe ergänzt werden kann.
Diese weiteren Einsatzstoffe sind:
- Pflanzenextrakt (natürlich) Capsaicin = Cayennepfeffer-Extrakt,
- natürliche und synthetische Duftstoffe sowie
- sonstige Zusätze, wie wertvolle Hautpflege-Öle (pflanzlich).

### Ausgangsmaterial ist die Algenfrischmasse (Schritt 4) pH 8,5

Pulvrige Löss- und Cellulose-Zusätze variieren je nach Lagerungs-Verfahren:

**Zusatzstoff-Mischung I: Wickel Kurzzeit-Lagerung = Kühlschrank 2-4°C**

| | |
|---|---|
| 20 - 30 % | Löss-Erde |
| 5 - 7,5 % | Cellulose |

**Zusatzstoff-Mischung II: Wickel Langzeit-Lagerung = Gefrierschrank- 25°C**

| | |
|---|---|
| 30,0 - 40 % | Löss-Erde |
| 7,5 - 10 % | Cellulose. |

Die Zugabe von Pflanzenextrakt Capsaicin hängt von der Größe des Wickels ab:

| | |
|---|---|
| Größe L (Large) | 3 Tropfen Capsaicin |
| Größe M (Middle) | 2 Tropfen Capsaicin |
| Größe S (Small) | 1 Tropfen Capsaicin. |

Natürliche Duftstoffe sind Arnika und Melisse.
Synthetische Duftstoffe sind (Fa. Bell): Honey, Green Tea, Sandelholz, Aloe Vera.

Zuerst wird die Heilerde, dann die Cellulose in die frische Biomasse (Schritt 3) jeweils solange schonend eingerührt (Rührgerät) bis eine homogene Masse resultiert. Pflanzenextrakte und andere Zusätze können dann durch nochmaliges vorsichtiges Rühren in der pastösen Masse gleichmäßig verteilt werden. Diese fertige Wickelmasse ist die Grundlage für den Algenwickel und zugleich als Algenpaste verkaufsfähig.
Natürliche / synthetische Duftstoffe werden anwendungsorientiert eingesetzt. D. h. Arnika, Honigtraum oder Aloe Vera verleihen dem wärmenden Algenwickel eine warme, angemessene Duftkomponente während Green Tea oder Sandelholz für eine frische Duftkomponente im kühlenden Wickel sorgen. Sonstige Zusätze wie Öle pflanzlichen Ursprungs haben eine hautpflegende Wirkung.

### Ausführungsbeispiel 4

### Algenprodukt ausschließlich aus einer Algenfrischmasse

Ist die Algenfrischmasse das Algenprodukt, so ist es zweckmäßig, dass dafür nur die frisch geerntete Spirulina verwendet wird. Lediglich der pH-Wert, der ursprünglich um 9,5 liegt, ist mit 20 %iger Ascorbinsäure-Lösung auf 8,2 - 8,0 einzustellen.

Soll die Spirulina als Frischmasse auf die Haut aufgetragen werden, wird sie vom stark basischen Milieu auf den hautverträglichen pH Bereich von 8,2 - 8,0 gesenkt.

## Patentansprüche

1. Verfahren zur Herstellung einer Algenfrischmasse, wobei nach der Ernte unter Nutzung eines Sieb-Filtrationsverfahrens flüssiges Algensubstrat mit einem Trockensubstanzgehalt von 5 bis 7 % und einem pH-Wert zwischen 9,2 bis 9,6 vorliegt, **dadurch gekennzeichnet, dass** nach der Ernte eine Lagerung des flüssigen Algensubstrates, wobei die Algenzelle des flüssigen Algensubstrates auch nach der Ernte lebens- und damit teilungsfähig bleibt, bis zu 48 Stunden im Prozessmedium unter Prozessbedingungen, nämlich bei einer Temperatur von 25-30°C und natürlicher Beleuchtung oder bei einer Temperatur von 2- 4°C und ohne Beleuchtung, erfolgt und das frisch geerntete flüssige Algensubstrat und/oder das bis zu 48 Stunden gelagerte flüssige Algensubstrat zu einer Algenfrischmasse verarbeitet werden, wobei das flüssige Algensubstrat auf einen Trockensubstanzgehalt von 10 bis 15% entwässert und nachfolgend der pH-Wert zwischen 8,5 bis 8,8 eingestellt wird.

2. Verfahren gemäß Anspruch 1, **dadurch gekennzeichnet, dass** nachfolgend eine Verarbeitung zu einem Algenprodukt erfolgt.

3. Verfahren gemäß Anspruch 1, **dadurch gekennzeichnet, dass** während der Ernte der lebenden Frischmasse eine Abtrennung von flüssigem Algensubstrat, bevorzugt von flüssigem Algensubstrat mit Spirulina-Algen mit einer Ausdehnung von zumindest 30 µm, von dem Gemisch aus kleinen Spirulina-Algen und Fremdalgen mit jeweils einer maximalen Ausdehnung von 10 µm erfolgt.

4. Verfahren gemäß Anspruch 2, **dadurch gekennzeichnet, dass** nach der Lagerung des flüssigen Algensubstrates dieses flüssige Algensubstrat auf einen Trockensubstanzgehalt von 10 bis 13 % entwässert wird.

5. Verfahren gemäß Anspruch 1, **dadurch gekennzeichnet, dass** der pH-Wert des flüssigen Algensubstrates durch Zugabe einer Ascorbinsäure-Lösung, bevorzugt einer 20 % -igen Ascorbinsäure-Lösung, auf einen Wert zwischen 8,2 bis 8,8 eingestellt wird.

## Claims

1. Procedure for the production of a fresh algae mass, with there being liquid algae substrate with a try substance content of 5 to 7 % and a pH value between 9.2 and 9.6 after harvesting using a screen filtration method, **characterized in that** storage of the liquid algae substrate, in which the algae cell of the liquid algae substrate remains vital and thus able to divide even after the harvest, for up to 48 hours in the process medium under process conditions after harvest, specifically at a temperature of 25-30 °C and natural lighting or at a temperature of 2-4 °C and without lighting, and the freshly harvested liquid algae substrate and/or the liquid algae substrate stored for up to 48 hours being processed into a fresh algae mass, with the liquid algae substrate being drained to a dry substance content of 10 to 15% and the pH-value being subsequently set to 8.5 to 8.8.

2. Procedure according to claim 1, **characterized In that** processing into an algae product takes place subsequently.

3. Procedure according to claim 1, **characterized In that** separation of liquid algae substrate, preferably of liquid algae substrate with spirulina algae with an expansion of at least 30 mm, from the mix of small spirulina algae and other algae, each with a maximum expansion of 10 mm, takes place while harvesting the living fresh mass.

4. Procedure according to claim 2, **characterized In that** this liquid algae substrate Is drained to a dry substance content of 10 to 13 % after storage of the liquid algae substrate.

5. Procedure according to claim 1, **characterized in that** the pH-value of the liquid algae substrate is adjusted to a value between 8.2 and 8.8 by addition of an ascorbic acid solution, preferably a 20-%-ascorbic acid solution.

## Revendications

1. Procédé de production d'une masse d'algues fraîches, un substrat d'algues liquide ayant une teneur en matière sèche de 5 à 7 % et un pH de 9,2 à 9,6 étant présent après la récolte en utilisant un procédé de filtrage par tamis, **caractérisé en ce que**, après la récolte, un stockage du substrat d'algues liquide, la cellule d'algues du substrat d'algues liquide restant viable et capable de se diviser même après la récolte, se produisant jusqu'à 48 heures dans le milieu de processus dans des conditions de processus, à savoir à une température de 25-30 ° C et sous un éclairage naturel ou à une température de 2-4 ° C et sans éclairage et le substrat d'algues liquide fraîchement récolté
et/ou le substrat d'algues liquide stocké jusqu'à 48 heures étant transformé en une masse d'algues fraîches, le substrat d'algues liquide étant déshydraté à une teneur en matière sèche de 10 à 15 % et ensuite le pH étant ajusté entre 8,5 et 8,8.

2. Procédé selon la revendication 1, **caractérisé en ce que** le traitement est ensuite transformé en un produit à base d'algues.

3. Procédé selon la revendication 1, **caractérisé en ce que** lors de la récolte de la masse fraîche vivante, il se produit une séparation d'un substrat d'algues liquide,
de préférence d'un substrat d'algues liquides avec des algues de Spiruline ayant une extension d'au moins 30 µm, du mélange de petites algues de Spiruline et d'algues étrangères avec, respectivement une extension maximale de 10 µm.

4. Procédé selon la revendication 2, **caractérisé en ce que**, après stockage du substrat d'algues liquide, ce substrat d'algues liquide est déshydraté à une teneur en matière sèche de 10 à 13 %.

5. Procédé selon la revendication 1, **caractérisé en ce que** le pH du substrat d'algues liquide en ajoutant une solution d'acide ascorbique, de préférence une solution d'acide ascorbique à 20 %, est ajusté à une valeur comprise entre 8,2 et 8,8.
